(19) Europäisches Patentamt
European Patent Office
Office européen des brevets

(11) Veröffentlichungsnummer: **0 482 543 B1**

# (12) EUROPÄISCHE PATENTSCHRIFT

(45) Veröffentlichungstag der Patentschrift: **02.08.95**

(21) Anmeldenummer: **91117905.9**

(22) Anmeldetag: **21.10.91**

(51) Int. Cl.⁶: **C07H 17/04**, C07H 17/08, C12P 19/60, C12N 1/20, //(C12N1/20,C12R1:465,1:625)

## (54) Oasomycine, Verfahren zu ihrer Herstellung und ihre Verwendung.

(30) Priorität: **26.10.90 DE 4034110**
**15.11.90 DE 4036360**

(43) Veröffentlichungstag der Anmeldung:
**29.04.92 Patentblatt 92/18**

(45) Bekanntmachung des Hinweises auf die Patenterteilung:
**02.08.95 Patentblatt 95/31**

(84) Benannte Vertragsstaaten:
**AT BE CH DE DK ES FR GB GR IT LI LU NL SE**

(56) Entgegenhaltungen:
**EP-A- 0 272 668**
**EP-A- 0 357 226**
**EP-A- 0 431 874**
**GB-A- 2 053 214**

**JOURNAL OF ANTIBIOTICS vol. 33, no. 9, 1980, TOKYO JP, pages 998-1004; J Pawlak et al.:"The structure of Lienomycin, a Pentaene Macrolide Anti-tumour Antibiotic. II. The location of the Pentaene Chromophore and of six isolated double bonds. The complete structure of the antibiotic"**

(73) Patentinhaber: **HOECHST AKTIENGESELLSCHAFT**
**D-65926 Frankfurt (DE)**

(72) Erfinder: **Zeeck, Axel, Prof. Dr.**
**Brüder-Grimm-Allee 22**
**W-3400 Göttingen (DE)**
Erfinder: **Philipps, Siegrid, Dr.**
**Bultstrasse 12**
**W-3100 Celle (DE)**
Erfinder: **Grabley, Susanne, Dr.**
**Hölderlinstrasse 7**
**W-6240 Königstein/Taunus (DE)**
Erfinder: **Granzer, Ernold, Dr. Dr.**
**Falkensteiner Strasse 24**
**W-6233 Kelkheim/Taunus (DE)**
Erfinder: **Hütter, Klaus, Dr.**
**Robert-Stolz-Strasse 3**
**W-6232 Bad Soden am Taunus (DE)**
Erfinder: **Thiericke, Ralf, Dr.**
**Auestrasse 35**
**W-6057 Dietzenbach (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 482 543 B1

Erfinder: **Wink, Joachim, Dr.**
**Magdeburger Strasse 14**
**W-6074 Rödermark (DE)**

## Beschreibung

Antibiotika aus Streptomyceten sind seit langem bekannte Wirkstoffe aus Mikroorganismen, die auf vielfältige Weise zu verwenden sind. J.V. Uri (Acta Microbiologica 33, 271 (1986)) beschreibt nicht polygene Makrolid-Antibiotika aus Streptomyceten, die Desertomycine. Diese Verbindungen besitzen ein breites antibakterielles Spektrum und selektive antifungische Aktivität. Es wurde gefunden, daß Streptoverticillium- und Streptomyces-Stämme neue makrocyclische Lactone, die Oasomycine, produzieren können. Diese Verbindungen besitzen pharmakologische und damit therapeutische Wirksamkeit und können insbesondere vorteilhaft als antibakterielles Mittel sowie als Inhibitor der Cholesterin-Biosynthese mit entsprechendem pharmakologischen Nutzen eingesetzt werden.

Die Erfindung betrifft somit:

1. Eine Verbindung der allgemeinen Formel I,

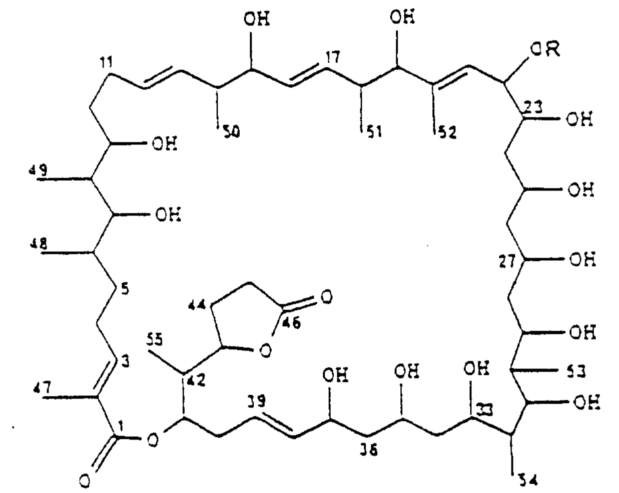

I

in der
R Wasserstoff oder die Gruppe

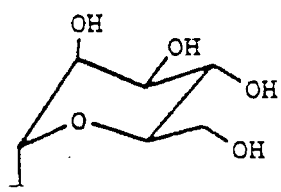

bedeutet,
sowie ein Verfahren zu deren Herstellung, das dadurch gekennzeichnet ist, daß Streptoverticilium sp. DSM 5989 und/oder Streptomyces sp. DSM 5990 in einem Nährmedium kultiviert werden, bis sich die Verbindung der allgemeinen Formel I in der Kultur anhäuft.

2. Verwendung der Verbindung der allgemeinen Formel I für die Herstellung eines Arzneimittels zur Prophylaxe und Behandlung von Krankheiten, die auf einem erhöhten Cholesterinspiegel beruhen.

3. Verwendung der Verbindung der allgemeinen Formel I für die Herstellung eines Arzneimittels zur Prophylaxe und Behandlung von bakteriellen Infektionen und Infektionskrankheiten.

Im folgenden wird die Erfindung detailliert beschrieben, insbesondere in ihren bevorzugten Ausführungsformen. Ferner wird die Erfindung in den Patentansprüchen definiert.

Die erfindungsgemäße Verbindung wird durch Kultivierung von Streptoverticillium sp. DSM 5989 und Streptomyces sp. DSM 5990 hergestellt. Beide Stämme wurden aus Erdproben aus Indien isoliert und bei der Deutschen Sammlung von Mikroorganismen nach den Regeln des Budapester Vertrages am 18.06.1990 unter obengenannter Nummer hinterlegt.

Der Stamm Streptoverticillium sp. DSM 5989 besitzt rote Sporen. Die Sporenketten sind verticilliert und ihre Oberfläche ist glatt. Der Stamm Streptomyces sp. DSM 5990 besitzt weiße Sporenketten, die in engen

Spiralen angeordnet sind. Die Oberfläche die Sporen erscheint glatt.

In einer Nährlösung, die eine Kohlenstoffquelle und eine Stickstoffquelle sowie die üblichen anorganischen Salze enthält, produzieren Streptoverticillium sp. und Streptomyces sp., bevorzugt DSM 5989 und DSM 5990, die Verbindung der allgemeinen Formel I. Anstelle der Stämme DSM 5989 oder DSM 5990 können natürlich auch deren Mutanten und Varianten eingesetzt werden, soweit sie diese Verbindung synthetisieren. Solche Mutanten können in an sich bekannter Weise durch physikalische Mittel, beispielsweise Bestrahlung, wie mit Ultraviolet- oder Röntgenstrahlen, oder chemische Mutagene, wie beispielsweise Ethylmethansulfonat (EMS), 2-Hydroxy-4-methoxy-benzophenon (MOB) oder N-Methyl-N'-nitro-N-nitrosoguanidin (MNNG) erzeugt werden.

Als bevorzugte Kohlenstoffquellen für die aerobe Fermentation eignen sich assimilierbare Kohlenhydrate und Zuckeralkohole, wie Glukose, Laktose oder D-Mannit sowie kohlenhydrathaltige Naturprodukte, wie Malzextrakt. Als stickstoffhaltige Nährstoffe kommen in Betracht: Aminosäuren, Peptide und Proteine sowie deren Abbauprodukte, wie Peptone oder Tryptone, ferner Fleischextrakte, gemahlene Samen, beispielsweise von Mais, Weizen, Bohnen, Soja oder der Baumwollpflanze, Destillationsrückstände der Alkoholherstellung, Fleischmehle oder Hefeextrakte, aber auch Ammoniumsalze und Nitrate. An anorganischen Salzen kann die Nährlösung beispielsweise Chloride, Carbonate, Sulfate oder Phosphate der Alkali- oder Erdalkalimetalle, Eisen, Zink, Kobalt und Mangan enthalten.

Die Bildung der Verbindung der Formel I verläuft besonders gut in einer Nährlösung, die etwa 0,2 bis 5 %, bevorzugt 1 bis 4 %, Sojamehl und 0,2 bis 5 %, bevorzugt 1 bis 4 %, Mannit enthält, jeweils bezogen auf das Gewicht der gesamten Nährlösung. Ähnlich gute Ergebnisse erhält man auch mit einer Nährlösung, die 0,2 bis 5 %, bevorzugt 1 bis 3 %, Haferflocken und 0,1 bis 10 ml, bevorzugt 1 bis 5 ml, einer wässrigen Spurenelementlösung, bestehend aus 0,1 bis 10 % Calciumchlorid, 0,01 bis 5 % Eisen-III-citrat, 0,01 bis 0,1 % Mangansulfat, 0,001 bis 0,5 % Zinkchlorid, 0,0001 bis 0,1 % Kupfersulfat, 0,001 bis 0,5 % Natriumtetraborat, 0,0001 bis 0,01 % Kobaltchlorid und 0,0001 bis 0,01 % Natriummolybdat, enthält sowie einer Nährlösung, die etwa 0,2 bis 10 %, bevorzugt 1 bis 5 %, Glycerin, 0,01 bis 1 %, bevorzugt 0,1 bis 0,4 %, Caseinpepton, 0,01 bis 1 %, bevorzugt 0,05 bis 0,5 %, Kaliumhydrogenphosphat, 0,001 bis 2 %, bevorzugt 0,01 bis 0,8 %, Natriumchlorid, 0,0001 bis 0,5 %, bevorzugt 0,001 bis 0,2 %, Magnesiumsulfat und 0,1 bis 20 ml, bevorzugt 1 bis 10 ml, einer wäßrigen Spurenelementlösung, bestehend aus 0,1 bis 10 % Calciumchlorid, 0,01 bis 5 % Eisen-III-citrat, 0,01 bis 0,1 % Mangansulfat, 0,001 bis 0,5 % Zinkchlorid, 0,0001 bis 0,1 % Kupfersulfat, 0,001 bis 0,5 % Natriumtetraborat, 0,0001 bis 0,01% Kobaltchlorid und 0,0001 bis 0,01 % Natriummolybdat, enthält, jeweils bezogen auf das Gewicht der gesamten Nährlösung. Die Kultivierung erfolgt aerob, also beispielsweise submers unter Schütteln oder Rühren in Schüttelkolben oder Fermentern, gegebenenfalls unter Einführen von Luft oder Sauerstoff. Sie kann in einem Temperaturbereich von etwa 18 bis 35 °C, vorzugsweise bei etwa 25 bis 35 °C, insbesondere bei 28 bis 32 °C durchgeführt werden. Der pH-Bereich sollte zwischen 6 und 8 liegen, vorteilhaft zwischen 6,5 und 7,5. Man kultiviert den Mikroorganismus unter diesen Bedingungen im allgemeinen über einen Zeitraum von 24 bis 300 Stunden, bevorzugt 36 bis 140 Stunden.

Vorteilhaft kultiviert man in mehreren Stufen, d. h. man stellt zunächst eine oder mehrere Vorkulturen in einem flüssigen Nährmedium her, die dann in das eigentliche Produktionsmedium, die Hauptkultur, beispielsweise im Volumenverhältnis 1:10, überimpft werden. Die Vorkultur erhält man z. B., indem man ein versportes Mycel in eine Nährlösung überimpft und etwa 36 bis 120 Stunden, bevorzugt 48 bis 72 Stunden, wachsen läßt. Das versporte Mycel kann beispielsweise erhalten werden, indem man den Stamm etwa 3 bis 40 Tage, bevorzugt 4 bis 10 Tage, auf einem festen oder flüssigen Nährboden, beispielsweise Hefe-Malz-Agar oder Sojamehl-Mannit-Agar, wachsen läßt.

Der Fermentationsverlauf kann anhand des pH-Wertes der Kultur oder des Mycelvolumens sowie durch chromatographische Methoden, wie z. B. Dünnschichtchromatographie oder High Pressure Liquid Chromatography, oder ausprüfen der biologischen Aktivität überwacht werden. Die Verbindung der allgemeinen Formel I ist sowohl im Mycel als auch im Kulturfiltrat enthalten.

Die Isolierung der genannten Verbindung aus dem Kulturmedium erfolgt nach bekannten Methoden unter Berücksichtigung der chemischen, physikalischen und biologischen Eigenschaften der Produkte. Zum Testen der Antibiotika-Konzentration im Kulturmedium oder in den einzelnen Isolierungsstufen kann die Dünnschichtchromatographie, beispielsweise an Kieselgel mit Butanol/Eisessig/Wasser oder Ethylacetat/Methanol/Wasser Mischungen als Laufmittel verwendet werden. Die Detektion bei der dünnschichtchromatographischen Auftrennung kann beispielsweise durch Färbereagentien wie Anisaldehyd oder durch biologische Testung, z. B. mit Bakterien, Pilzen oder Protozoen erfolgen, wobei die Menge der gebildeten Substanz zweckmäßig mit einer Eichlösung verglichen wird.

Zur Isolierung der Verbindung I werden Kulturbrühe und Mycel zuerst mit unpolaren organischen Lösungsmitteln, wie z. B. n-Hexan, Petrolether oder halogenierten Kohlenwasserstoffen wie z. B. Chloroform

usw. extrahiert, um die unpolaren Verunreinigungen zu entfernen. Anschließend wird mit einem polareren organischen Lösungsmittel, beispielsweise niederen Alkoholen, Aceton und/oder Essigester sowie Gemischen dieser Lösungsmittel, extrahiert.

Die Reinisolierung der Verbindung der Formel I erfolgt an geeigneten Materialien, vorzugsweise z. B. an Kieselgel, Aluminiumoxid, Ionenaustauschern oder Adsorberharzen, durch anschließende Elution mit organischen, polaren Lösungsmitteln oder Lösungsmittelgemischen, wie z. B. Essigsäurealkylester, Gemische aus Essigsäurealkylester mit einem niederen Alkanol, Chloroform oder Methylenchlorid bzw. Gemischen dieser Lösungsmittel mit niederen Alkanolen, gegebenenfalls auch mit Wasser, bzw. einem für Ionenaustauscher-Harzen geeigneten pH- bzw. Salzgradienten, wie beispielsweise Kochsalz oder Tris-(hydroxymethyl)-aminomethan-HCl(Tris-Puffer), und Vereinigung der antibiotisch aktiven Fraktionen.

Die Verbindung der Formel I ist im festen Zustand und in Lösungen im pH-Bereich zwischen 1 und 8, insbesondere 3 und 7, stabil und läßt sich damit in übliche galenische Zubereitungen einarbeiten.

Die erfindungsgemäße Verbindung kann als antibakterielles Agens sowie auch als Lipidregulator verwendet werden. Insbesondere die Verbindung der Formel I, in der R Wasserstoff ist, läßt sich vorteilhaft als Lipidregulator, insbesondere zur Hemmung der Cholesterinbiosynthese verwenden.

Die Verbindung der allgemeinen Formel I kann zur Prophylaxe und Behandlung von Krankheiten verwendet werden, die auf einem erhöhten Cholesterinspiegel beruhen, insbesondere koronare Herzkrankheiten, Arteriosklerose und ähnlicher Krankheiten. Die Anwendung betrifft auch pharmazeutische Zubereitungen der Verbindung der Formel I.

Bei der Herstellung von Arzneimitteln können neben dem Wirkstoff auch pharmazeutisch unbedenkliche Zusatzstoffe, wie Verdünnungsmittel und/oder Trägermaterialien, verwendet werden. Hierunter sind physiologisch unbedenkliche Substanzen zu verstehen, die nach Vermischen mit dem Wirkstoff diesen in eine für die Verabreichung geeignete Form bringen.

Geeignete feste oder flüssige galenische Zubereitungsformen sind beispielsweise Tabletten, Dragees, Pulver, Kapseln, Suppositorien, Sirupe, Emulsionen, Suspensionen, Tropfen oder injizierbare Lösungen sowie Präparate mit protrahierter Wirkstoff-Freigabe. Als häufig verwendete Trägerstoffe bzw. Verdünnungsmittel seien z. B. verschiedene Zucker oder Stärkearten, Cellulosederivate, Magnesiumcarbonat, Gelatine, tierische und pflanzliche Öle, Polyethylenglykole, Wasser oder andere geeignete Lösungsmittel sowie wasserhaltige Puffermittel, die durch Zusatz von Glucose oder Salzen isotonisch gemacht werden können, genannt.

Außerdem können gegebenenfalls oberflächenaktive Mittel, Farb- und Geschmacksstoffe, Stabilisatoren, sowie Konservierungsmittel als weitere Zusatzstoffe in den erfindungsgemäßen Arzneimittelzubereitungen Verwendung finden. Es können auch pharmakologisch akzeptable polymere Träger, wie z.B. Polyphenylpyrolidone, oder andere pharmazeutisch akzeptable Additive, wie z.B. Cyclodextrin oder Polysaccharide, verwendet werden. Die Verbindungen können insbesondere auch mit Additiven, die Gallensäure binden, im besonderen nicht toxischen, im Gastrointestinal-Trakt nicht resorbierbaren, basischen Anionen-Austauschern, kombiniert werden.

Die Präparate können oral, rektal oder parenteral verabreicht werden. Vorzugsweise können die Präparate in Dosierungseinheiten hergestellt werden; insbesondere Tabletten, Kapseln, Suppositorien, stellen Beispiele für geeignete Dosierungseinheiten dar. Jede Dosierungseinheit, insbesondere für die orale Applikation, kann bis zu 1000 mg, bevorzugt jedoch 10 bis 100 mg, des aktiven Bestandteiles enthalten. Jedoch können auch darüber- oder darunterliegende Dosierungseinheiten verwendet werden, die gegebenenfalls vor Verabreichung zu teilen bzw. zu vervielfachen sind.

Gegebenenfalls können die Dosierungseinheiten für die orale Verabreichung mikroverkapselt werden, um die Abgabe zu verzögern, oder über einen längeren Zeitraum auszudehnen, wie beispielsweise durch Überziehen oder Einbetten des Wirkstoffs in Teilchenform in geeignete Polymere, Wachse oder dergleichen.

Die parenterale Verabreichung kann unter Verwendung flüssiger Dosierungsformen, wie steriler Lösungen und Suspensionen, erfolgen, die für die intramuskuläre oder subcutane Injektion bestimmt sind. Derartige Dosierungsformen werden durch Lösen bzw. Suspendieren einer abgemessenen Wirkstoffmenge in einem geeigneten physiologisch unbedenklichen Verdünnungsmittel wie beispielsweise einem wäßrigen oder öligen Medium und Sterilisierung der Lösung bzw. der Suspension gegebenenfalls unter Mitverwendung von geeigneten Stabilisatoren, Emulgatoren und/oder Konservierungsmitteln und/oder Antioxidantien hergestellt.

Die orale Applikationsform ist, insbesondere unter dem Gesichtspunkt einer langen Therapiedauer, bevorzugt und stellt eine wesentliche Erleichterung in der Prävention und Therapie oben angeführter Krankheiten dar.

Die pharmazeutischen Präparate werden nach allgemein üblichen Verfahren hergestellt. Das Dosierungsschema kann vom Typ, Alter, Gewicht, Geschlecht und medizinischen Zustand des Patienten oder Risikogefährdeten abhängen.

In den folgenden Beispielen wird die Erfindung in weiteren Details beschrieben. Prozentangaben beziehen sich auf das Gewicht, wenn nicht anders angegeben.

Beispiele:

1.

a) Herstellung einer Sporensuspension des Produzentenstammes:

100 ml Nährlösung [12,5 g Glycerin, 1 g Arginin, 1 g NaCl, 1 g Di-Kaliumhydrogenphosphat, 0,5g Magnesiumsulfat, 2,5 ml Spurenelementlösung A (3 g Calciumchlorid, 1 g Eisen-III-citrat, 0,2 g Mangansulfat, 0,1 g Zinkchlorid, 0,025 g Kupfersulfat, 0,02 g Natriumtetraborat, 0,004 g Kobaltchlorid, 0,01 g Natriummolybdat in 1 destilliertez Wasser), mit Wasser auf 1 l auffüllen, pH-wert vor der Sterilisation 7, 3] in einem 500 ml sterilen Erlenmeyerkolben werden mit dem Stamm DSM 5989 oder DSM 5990 beimpft und 72 Stunden bei 28 °C und 150 UpM auf einer rotierenden Schüttelmaschine inkubiert. Anschließend werden 20 ml Kulturflüssigkeit in einem sterilen 500 ml Erlenmeyerkolben mit dem Nährboden der obengenannten Zusammensetzung, dem zusätzlich 20 g Agar/l zur Verfestigung zugegeben wurde, gleichmäßig verteilt und dekantiert. Die Kulturen werden 10 bis 14 Tage bei 30 °C inkubiert. Die nach dieser Zeit entstandenen Sporen eines Kolbens werden mit 500 ml entionisiertem Wasser, das einen Tropfen eines handelsüblichen nichtionischen Tensids (z. B. Triton X 100, Fa. Serva) enthält, abgeschwemmt, sofort weiterverwendet oder bei -22 °C in 50 % Glycerin aufbewahrt.

b) Herstellung einer Kultur bzw. einer Vorkultur des Produzentenstammes im Erlenmeyerkolben:

Ein steriler 500 ml Erlenmeyerkolben mit 100 ml der unter a) beschriebenen Nährlösung wird mit einer auf einem Schrägröhrchen gewachsenen Kultur oder mit 0,2 ml Sporensuspension angeimpft und auf einer Schüttelmaschine bei 150 UpM und 30 °C inkubiert. Die maximale Produktion der Verbindung der Formel I ist nach ca. 72 Stunden erreicht. Zum Animpfen von 10 und 100 l Fermentern genügt eine 48 Stunden alte Submerskultur (Animpfmenge ca. 5 %) aus der gleichen Nährlösung.

2. Herstellung der Verbindung der allgemeinen Formel I:

Ein 10 l Fermenter wird unter folgenden Bedingungen betrieben:

| | |
|---|---|
| Nährmedium: | 20 g/l Sojamehl<br>20 g/l Mannit<br>pH 7,5 (vor der Sterilisation) |
| Inkubationszeit:<br>Inkubationstemperatur:<br>Rührergeschwindigkeit:<br>Belüftung: | 72 Stunden<br>30 °C<br>250 UpM<br>4 l Luft/min. |

Durch wiederholte Zugabe weniger Tropfen ethanolischer Polyollösung kann die Schaumbildung unterdrückt werden. Das Produktionsmaximum wird nach ca. 72 Stunden erreicht. Die Ausbeuten liegen bei ca. 100 mg/l.

3. Isolierung der Verbindung der allgemeinen Formel I:

Nach Beendigung der Fermentation von DSM 5989 bzw. DSM 5990 wird die Kulturbrühe unter Zusatz von ca. 2 % Filterhilfsmittel (z. B. Celite) filtriert. Es kann nach folgenden Schemata aufgearbeitet werden:

Aufarbeitung/Isolierung

Schema 1: Kulturüberstand

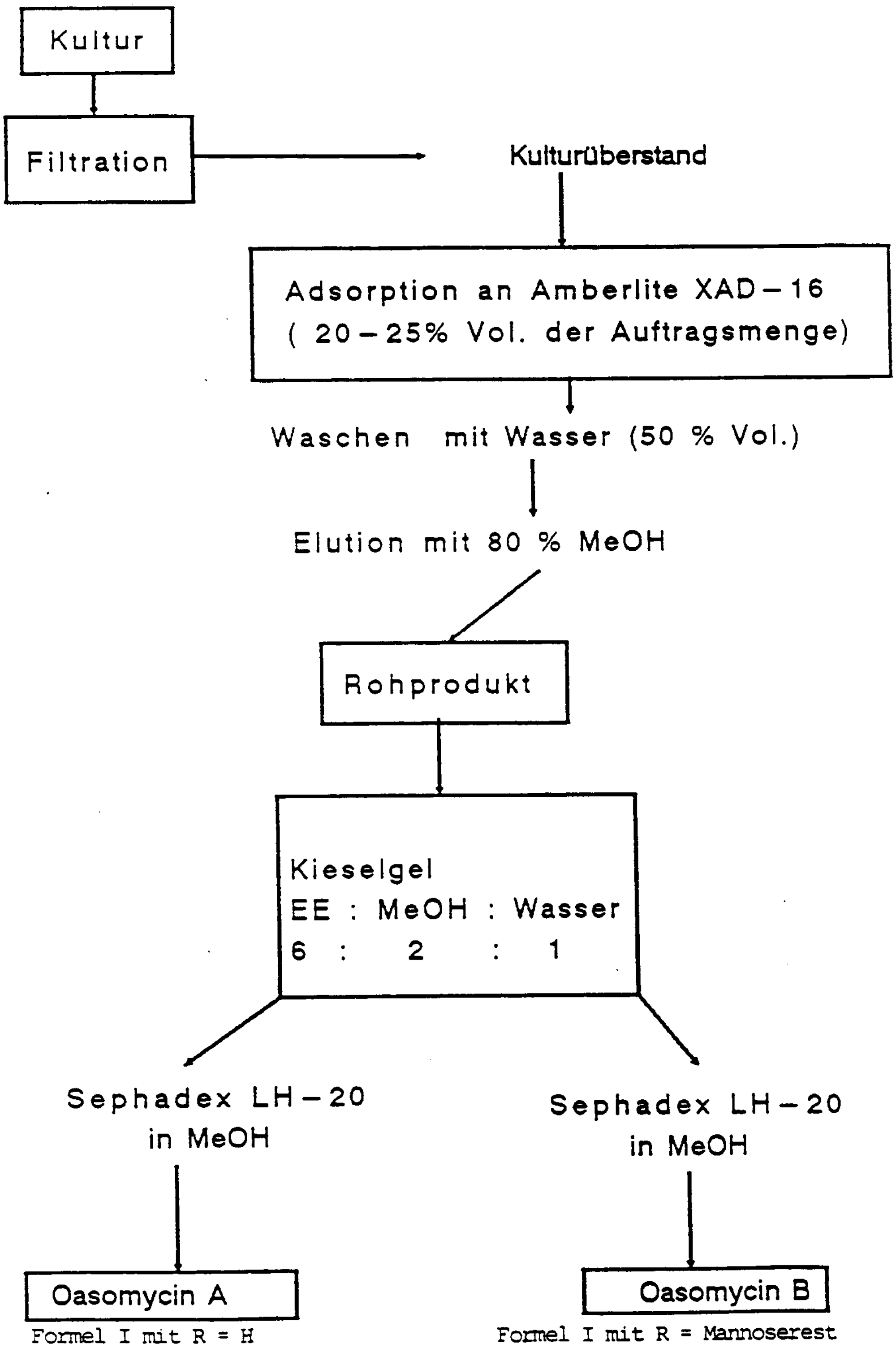

Aufarbeitung/Isolierung

Schema 1: Mycel

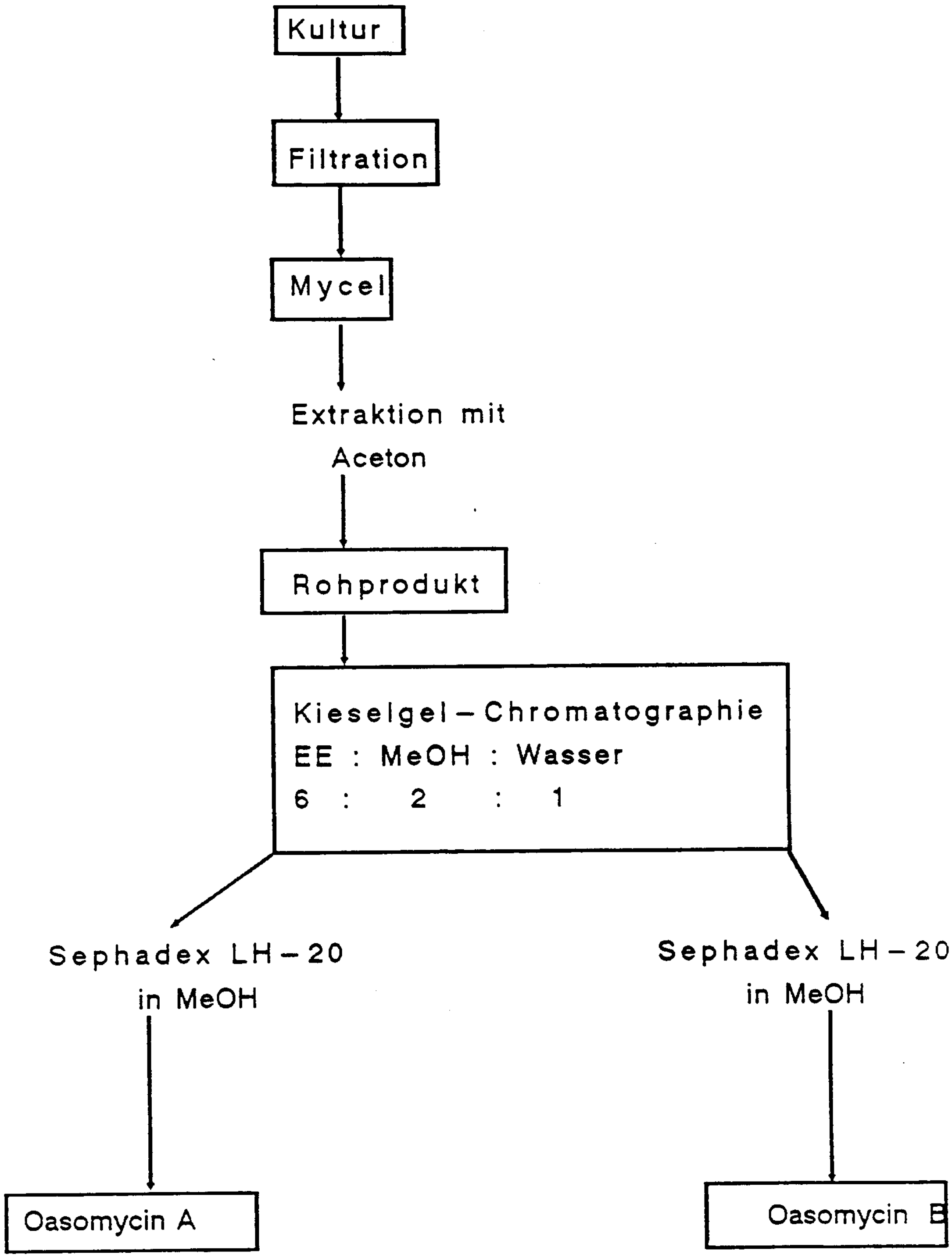


Formel I mit R = H

Formel I mit R = Mannoserest

4. Biologische Testung als Cholesterinbiosynthese-Inhibitor:

a) In Vitro-Bestimmung:

Monolayer von HEP-G2-Zellen in lipoproteinfreiem Nährmedium werden mit entsprechenden Konzentrationen der zu prüfenden Substanzen der allgemeinen Formel I eine Stunde vorinkubiert. Nach Zugabe der $^{14}C$-markierten Biosynthesevorstufe [$^{14}C$]Natriumacetat wird die Inkubation für 3 Stunden

fortgesetzt. Danach wird ein Teil der Zellen alkalisch verseift, bei vorheriger Zugabe eines internen Standards von $^{3}$H-Cholesterin. Die Lipide der verseiften Zellen werden mit einem Gemisch aus Chloroform-Methanol extrahiert. Dieses Lipidgemisch wird nach Zusatz von Trägercholesterin präparativ dünnschichtchromatographisch aufgetrennt, die Cholesterinbande nach Anfärbung isoliert und die aus dem $^{14}$C-Precursor gebildete Menge $^{14}$C-Cholesterin szintigraphisch bestimmt. In einem aliquoten Teil der Zellen wurde Zellprotein bestimmt, sodaß die in der Zeiteinheit pro mg Zellprotein aus $^{14}$C-Vorläufer gebildete Menge $^{14}$C-Cholesterins berechnet werden kann. Zum Vergleich für die Hemmwirkung eines zugesetzten Prüfpräparates dient die Kontrolle, so daß direkt die Hemmung der Cholesterinbiosynthese bei einer bestimmten molaren Konzentration des Prüfpräparates im Medium angegeben werden kann. In aliquoten Anteilen der Zellkultur wird die Intaktheit der Zellkultur und die fehlende Zellschädigung durch Präparateeinwirkung morphologisch (Lichtmikroskopie) beurteilt und biochemisch durch Bestimmung der Laktat-Dehydrogenase Ausschüttung in das Inkubationsmedium gemessen. Als Standartpräparat wurde Lov astatin benutzt. Die Verbindung der Formel I mit R = H hemmt die Cholesterin-Biosynthese in einer Konzentration von
$10^{-7}$ mol/l zu 72 %
$10^{-8}$ mol/l zu 41 %
Lovastatin hemmt die Cholesterin-Biosynthese bei $10^{-7}$ mol/l zu 74 % und bei
$10^{-8}$ mol/l zu 48 %.
b) In Vivo-Bestimmung:
Die Hemmung der hepatischen Cholesterinbiosynthese hat Auswirkungen auf die Verminderung von Serum-Lipiden, wie im chronischen Versuch an der männlichen Ratte nachgewiesen werden kann. Gruppen männlicher Ratten des Stammes HOE: WISKf (SPF 71) mit einem Ausgangsgewicht von etwa 240 g erhalten täglich morgens die Prüfpräparate in Polyethylenglykol 400 per Schlundsonde, wobei die jeweilige Kontrolgruppe nur das Vehikel erhielt. 24 Stunden nach der letzten Applikation und nach 24 Stunden Futterentzug wurde Blut entnommen und im gewonnenen Serum die Lipoproteine aus dem Pool einer Rattengruppe mittels der präparativen Ultrazentrifugen-Technik getrennt. Hierbei wurden folgende Dichtegrenzen für die Trennung von VLDL, LDL und HDL benutzt:
VLDL 1.006
LDL 1.04
HDL 1.21
Für die Bestimmung des Cholesterins und der Triglyceride wurden vollenzymatische Methoden nach Boehringer/Mannheim benutzt, für die Bestimmung des Proteins die Methode von Lowry et al. herangezogen.

Die gemessenen Werte für die Verbindung der Formel I, in der R Wasserstoff bedeutet im Vergleich zu Clofibrat, sind im folgenden aufgeführt:

Biologische Daten

| Verbindung | Dosis | Totalcholesterin | | | Protein | Glyz | | Cholesterin | | |
|---|---|---|---|---|---|---|---|---|---|---|
| | mg/kg | VDLD | | LDL | HDL | VLDL | | LDL | VLDL | HDL/LDL |
| Formel I | | | | | | | | | | |
| R = H | 20 | -30 | -45 | -4 | +2 | -32 | -6 | 1,74 | | |
| Clofibrat | 100 | -54 | -32 | -28 | -8 | -9 | -10 | 1,05 | | |

5.

a) Charakterisierung der Verbindung I, in der R Wasserstoff bedeutet:
Oasomycin A wurde als amorpher Feststoff isoliert.
Schmelzpunkt: 146 ° C
Drehwert:-13,1 ° (c = 0,122, Methanol)
Dünnschichtchromatographie:
Kieselgel 60, F254: Chloroform/Methanol (9:1, v:v): Rf 0,0
n-Butanol/Essigsäure/Wasser (Oberphase) (4:1:5, v:v:v): Rf 0,45

Anfärbeverhalten: Ehrlich-Reagenz: braun; Vanillin-Schwefelsäure: braun/violett.
FAB-MS: m/e = 1065 (M + K); 1049 (M + Na);
Molmasse: 1027,3 ($C_{55}H_{94}O_{17}$)
IR (KBr): 3420 (br), 2960, 2930, 1780, 1725, 1710, 1600, 1570 cm-1
UV (MeOH): = 202 nm ( = 14000); 220 (6100)
+ HCl: = 202 (16400); 220 (5700)
+ NaOH: = 225(7000)
1H-NMR (500 MHz, DMSO-$d_6$) $\delta$ = 0.86 (53-$H_3$), 0.78 (55-$H_3$), 0.64 (54-$H_3$), 0.64 (49-$H_3$), 1.54 (52-$H_3$), 1.77 (47-$H_3$), 0.78 (48-$H_3$), 0.86 (50-$H_3$), 0.99 (51-$H_3$), 1.88 und 2.16 (44-$H_2$), 2.16 (4-$H_2$), 2.44 (45-$H_2$), 1.90 und 2.18 (11-$H_2$), 1.20 und 1.50 (10-$H_2$), 2.32 (40-$H_2$), 1.26 und 1.54 (5-$H_2$), 1.52 (6-H), 2.18 (18-H), 1.46 (30-H), 2.14 (42-H), 1.26 (32-H), 1.42 (24-$H_2$9, 1.58 (8-H), 2.08 (14-H), 1.11 und 1.42 (34-$H_2$), 1.52 (28-$H_2$), 1.40 (26-$H_2$), 1.34 (36-$H_2$), 3.90 (25-H), 3.82 (35-H), 3.90 (27-H), 4.06 (33-H), 4.14 (37-H), 3.68 (31-H), 4.06 (23-H), 3.66 (9-H), 3.62 (29-H), 5.00 (41-H), 3.74 (22-H), 3.74 (15-H), 3.25 (7-H), 4.48 (43-H), 3.50 (19-H), 5.48 (39-H), 5.26 (21-H), 5.40 (12-H), 5.34 (16-H), 5.39 (13-H), 5.39 (17-H), 5.48 (38-H), 6.69 (3-H).
13C-NMR (125.7 MHz, DMSO-$d_6$)
$\delta$ = 9.4 (q, C-53), 9.6 (q, C-55), 10.5 (q, C-54), 11.1 (q, C-49), 11.5 (q, C-52), 12.1 (q, C-47), 12.2 q, C-48), 15.2 (q, C-50), 16.8 (q, C-51), 24.8 (t, C-44), 26.0 (t, C-4), 28.2 (t, C-45), 28.9 (t, C-11), 32.0 (t, C-10), 32.4 (t, C-40), 32.9 (t, C-5), 34.3 (d, C-6), 39.2 (d, C-18), 39.5 (d, C-30), 39.9 (d C-42), 40.1 (d, C-32), 40.4 (t, C-24), 41.4 (d, C-8), 42.2 (d, C-14), 42.2 (t, C-34), 42.2 (t, C-28), 45.6 (t, C-26), 45.7 (t, C-36), 63.7 (d, C-25), 63.8 (d, C-35), 66.7 (d, C-27), 66.8 (d, C-33), 66.8 (d, C-37), 70.8 (d, C-31), 70.9 (d, C-23), 72.0 (d, C-9), 72.8 (d, C-29), 73.0 (d, C-41), 74.3 (d, C-22), 74.3 (d, C-15), 74.6 (d, C-7), 80.4 (d, C-43), 81.0 (d, C-19), 122.4 (d, C-39), 126.8 (s, C-2), 126.9 (d, C-21), 129.5 (d, C-12), 130.9 (d, C-16), 132.5 (d, C-13), 132.7 (d, C-17), 138.1 (s, C-20), 138.2 (d, C-38), 142.5 (d, C-3), 166.4 (s, C-1), 176.7 (s, C-46).

| | | | |
|---|---|---|---|
| Elementaranalyse: | Berechnet:<br>Gefunden: | C 64,30<br>C 64,20 | H 9,22<br>H 9,22 |

b) Charakterisierung der Verbindung I, in der R ein Mannoserest ist:
Oasomycin B wurde als amorpher Feststoff isoliert.
Schmelzpunkt: 157 ° C
Drehwert: -24,6 (c = 0,199, Methanol)
Dünnschichtchromatographie:
Kieselgel 60, F254: Chloroform/Methanol (9:1, v:v): Rf 0,0
n-Butanol/Essigsäure/Wasser (Oberphase) (4:1:5, v:v:v): Rf 0,30
Anfärbeverhalten: Ehrlich-Reagenz: braun; Vanillin-Schwefelsäure: blau/violett.
FAB-MS: m/e = 1228 (M + K); 1212 (M-Na);
Molmasse: 1189,5 ($C_{61}H_{104}O_{22}$)
IR (KBr):3400 (br), 2960, 2930, 1770, 1725, 1710, 1580 cm-1
UV (MeOH): = 202 nm ( = 23100); 220 (10900)
+ HCl: = 202 (24600); 220 (10100)
+ NaOH: = 218 (14000)
1H-NMR (500 MHz, DMSO-$d_6$) $\delta$ = 0,90 (53-$H_3$), 0,82 (55-$H_3$), 0,68 (54-$H_3$), 0,68 (49-$H_3$), 1,63 (52-$H_3$), 1,80 (47-$H_3$), 0,82 (48-$H_3$), 0,90 (50-$H_3$), 1,02 (51-$H_3$), 1,94 und 2,18 (44-$H_2$), 2,18 (4-$H_2$), 2,53 (45-$H_2$), 1,96 und 2,21 (11-$H_2$), 1,24 und 1,50 (10-$H_2$), 2,36 (40-$H_2$), 1,32 und 1,50 (5-$H_2$), 1,57 (6-H), 2,24 (18-H), 1,48 (30-H), 2,18 (42-H), 1,44 (32-H), 1,30 (24-$H_2$), 1,62 (8-H), 2,12 (14-H), 1,23 (34-$H_2$), 1,59 (28-$H_2$), 1,42 (26-$H_2$), 1,30 (36-$H_2$), 3,48 und 3,68 (6'-$H_2$), 3,86 (25-H), 3,82 (35-H), 3,92 (27-H), 4,10 (33-H), 4,15 (37-H), 3,44 (4'-H), 3,82 (23-H), 3,54 (2'-H), 3,78 (31-H), 3,56 (3'-H), 3,64 (9-H), 3,60 (29-H), 5,02 (41-H), 3,40 (5'-H), 4,16 (22-H), 3,70 (15-H), 3,26 (7-H), 4,42 (43-H), 3,54 (19-H), 4,63 (1'-H), 5,20 (21-H), 5,52 (39-H), 5,40 (12-H), 5,37 (16-H), 5,42 (13-H), 5,48 (17-H), 5,52 (38-H), 6,72 (3-H).
13C-NMR (125,7 MHz, DMSO-$d_6$)
$\delta$ = 9,4 (q, C-53), 9,6 (q, C-55), 10,4 (q, C-54), 11,2 (q, C-49), 11,7 (q, C-52), 12,1 (q, C-47), 12,2 (q, C-48), 15,2 (q, C-50), 16,6 (q, C-51), 24,8 (t, C-44), 26,1 (t, C-4), 28,2 (t, C-45), 28,9 (t, C-11), 32,1 (t, C-10), 32,4 (t, C-40), 32,9 (t, C-5), 34,3 (d, C-6), 39,4 (d, C-18), 39,4 (d, C-30), 39,9 (d, C-42), 40,4 (d, C-32), 40,8 (t, C-24), 41,4 (d, C-8), 42,2 (d, C-14), 42,2 (t, C-34), 42,3 (t, C-28), 45,6 (t, C-26), 45,7 (t, C-36), 61,2 (t, C-6'), 63,4 (d, C-25), 63,8 (d, C-35), 66,7 (d, C-27), 66,8 (d, C-33), 66,8 (d, C-37), 67,2

(d, C-4'), 69,4 (d, C-23), 70,6 (d, C-2'), 70,7 (d, C-31), 70,9 (d, C-3'), 72,0 (d, C-9), 72,8 (d, C-29), 73,0 (d, C-41), 73,5 (d, C-5'), 73,7 (d, C-22), 74.4 (d, C-15), 74,6 (d, C-7), 80,4 (d, C-43), 80,7 (d, C-19), 95,9 (d, C-1'), 122,2 (d, C-21), 122,4 (d, C-39), 126,9 (s, C-2), 129,5 (d, C-12), 131,0 (d, C-16), 132,6 (d, C-13), 132,7 (d, C-17), 138,2 (d, C-38), 142,5 (d, C-3), 143,2 (s, C-20), 166,4 (s, C-1), 176,7 (s, C-46).

| | | | |
|---|---|---|---|
| Elementaranalyse: | Berechnet: | C 61,89 | H 8,81 |
| | Gefunden: | C 59,91 | H 8,85 |

**Patentansprüche**
**Patentansprüche für folgende Vertragsstaaten : AT, BE, CH, DE, DK, FR, GB, IT, LI, LU, NL, SE**

1. Verbindung der allgemeinen Formel I,

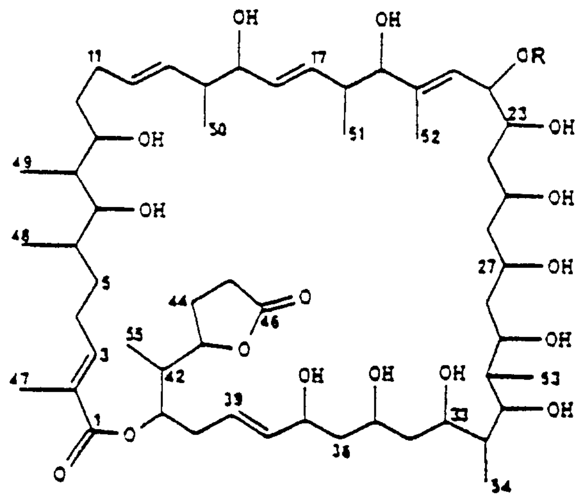

in der R Wasserstoff oder die Gruppe

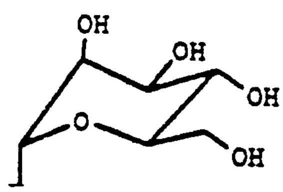

bedeutet.

2. Verfahren zur Herstellung der Verbindung der allgemeinen Formel I gemäß Anspruch 1, dadurch gekennzeichnet, daß die Stämme Streptoverticillium sp. DSM 5989 und/oder Streptomyces sp. DSM 5990 in einem Nährmedium kultiviert werden, bis sich die Verbindung der allgemeinen Formel I in der Kultur anhäuft.

3. Verfahren nach Anspruch 2, dadurch gekennzeichnet, daß das Nährmedium 0,2 bis 5 % Sojamehl und 0,2 bis 5 % Mannit enthält, jeweils bezogen auf das Gewicht der gesamten Nährlösung.

4. Verfahren nach Anspruch 3, dadurch gekennzeichnet, daß das Nährmedium 0,2 bis 5 % Haferflocken und 0,1 bis 10 ml einer wäßrigen Spurenelementlösung, bestehend aus 0,1 bis 10 % Calciumchlorid, 0,01 bis 5 % Eisen-III-citrat, 0,01 bis 0,1 % Mangansuslfat, 0,001 bis 0,5 % Zinkchlorid, 0,0001 bis 0,1 % Kupfersulfat, 0,001 bis 0,5 % Natriumtetraborat, 0,0001 bis 0,01 % Kobaltchlorid und 0,0001 bis 0,01

% Natriummolybdat, enthält.

5. Verfahren nach einem oder mehreren der Ansprüche 2 bis 4, dadurch gekennzeichnet, daß die Kultivierung bei 18 bis 35 °C erfolgt.

6. Verfahren nach einem oder mehreren der Ansprüche 2 bis 5, dadurch gekennzeichnet, daß die Kultivierung in dem pH-Bereich zwischen 6 und 8 erfolgt.

7. Arzneimittel, enthaltend die Verbindung der allgemeinen Formel I gemäß Anspruch 1 und gegebenenfalls pharmazeutisch unbedenkliche Zusatzstoffe.

8. Verwendung der Verbindung der allgemeinen Formel I gemäß Anspruch 1 für die Herstellung eines Arzneimittels zur Prophylaxe und Behandlung von Krankheiten, die auf einem erhöhten Cholesterinspiegel beruhen.

9. Verwendung nach Anspruch 8, dadurch gekennzeichnet, daß die Verbindung, in der R den Mannose-Rest darstellt, eingesetzt wird.

10. Verwendung der Verbindung der allgemeinen Formel I gemäß Anspruch 1 für die Herstellung eines Arzneimittels zur Prophylaxe und Behandlung von bakteriellen Infektionen und Infektionskrankheiten.

11. Streptoverticillium sp. DSM 5989 und Streptomyces sp. DSM 5990 sowie deren Varianten und Mutanten, soweit sie die Verbindung der allgemeinen Formel I gemäß Anspruch 1 herstellen.

**Patentansprüche für folgende Vertragsstaaten : ES, GR**

1. Verfahren zur Herstellung der Verbindung der allgemeinen Formel I

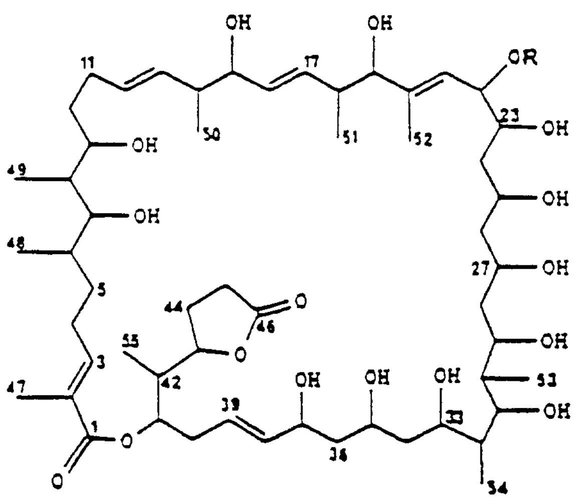

in der R Wasserstoff oder die Gruppe

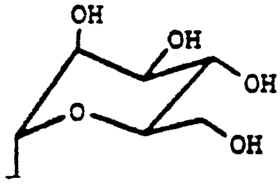

bedeutet,
dadurch gekennzeichnet, daß die Stämme Streptoverticillium sp. DSM 5989 und/oder Streptomyces sp. DSM 5990 in einem Nährmedium kultiviert werden, bis sich die Verbindung der allgemeinen Formel I in

der Kultur anhäuft.

2. Verfahren nach Anspruch 1, dadurch gekennzeichnet, daß das Nährmedium 0,2 bis 5 % Sojamehl und 0,2 bis 5 % Mannit enthält, jeweils bezogen auf das Gewicht der gesamten Nährlösung.

3. Verfahren nach Anspruch 2, dadurch gekennzeichnet, daß das Nährmedium 0,2 bis 5 % Haferflocken und 0,1 bis 10 ml einer wäßrigen Spurenelementlösung, bestehend aus 0,1 bis 10 % Calciumchlorid, 0,01 bis 5 % Eisen-III-citrat, 0,01 bis 0,1 % Mangansulfat, 0,001 bis 0,5 % Zinkchlorid, 0,0001 bis 0,1 % Kupfersulfat, 0,001 bis 0,5 % Natriumtetraborat, 0,0001 bis 0,01 Kobaltchlorid und 0,0001 bis 0,01 % Natriummolybdat, enthält.

4. Verfahren nach einem oder mehreren der Ansprüche 1 bis 3, dadurch gekennzeichnet, daß die Kultivierung bei 18 bis 35 °C erfolgt.

5. Verfahren nach einem oder mehreren der Ansprüche 1 bis 4, dadurch gekennzeichnet, daß die Kultivierung in dem pH-Bereich zwischen 6 und 8 erfolgt.

6. Verwendung der Verbindung der allgemeinen Formel I gemäß Anspruch 1 für die Herstellung eines Arzneimittels zur Prophylaxe und Behandlung von Krankheiten, die auf einem erhöhten Cholesterinspiegel beruhen.

7. Verwendung nach Anspruch 6, dadurch gekennzeichnet, daß die Verbindung, in der R den Mannose-Rest darstellt, eingesetzt wird.

8. Verwendung der Verbindung der allgemeinen Formel I gemäß Anspruch 1 für die Herstellung eines Arzneimittels zur Prophylaxe und Behandlung von bakteriellen Infektionen und Infektionskrankheiten.

9. Streptoverticillium sp. DSM 5989 und Streptomyces sp. DSM 5990 sowie deren Varianten und Mutanten, soweit sie die Verbindung der allgemeinen Formel I gemäß Anspruch 1 herstellen.

**Claims**

**Claims for the following Contracting States : AT, BE, CH, DE, DK, FR, GB, IT, LI, LU, NL, SE**

1. A compound of the formula I

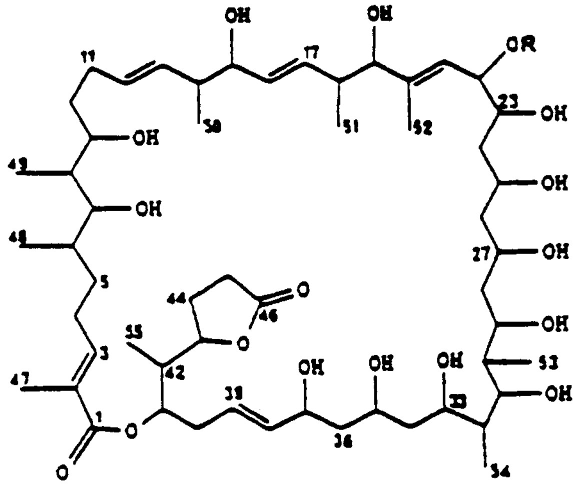

in which R is hydrogen or the group

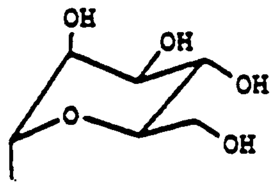.

2. A process for the preparation of the compound of the formula I as claimed in claim 1, which comprises cultivating the strains Streptoverticillium sp. DSM 5989 and/or Streptomyces sp. DSM 5990 in a nutrient medium until the compound of the formula I accumulates in the culture.

3. The process as claimed in claim 2, wherein the nutrient medium contains 0.2 to 5 % soybean meal and 0.2 to 5 % mannitol, in each case based on the weight of the complete nutrient solution.

4. The process as claimed in claim 3, wherein the nutrient medium contains 0.2 to 5 % rolled oats and 0.1 to 10 ml of an aqueous trace element solution composed of 0.1 to 10 % calcium chloride, 0.01 to 5 % iron(III) citrate, 0.01 to 0.1 % manganese sulfate, 0.001 to 0.5 % zinc chloride, 0.0001 to 0.1 % copper sulfate, 0.001 to 0.5 % sodium tetraborate, 0.0001 to 0.01 % cobalt chloride and 0.0001 to 0.01 % sodium molybdate.

5. The process as claimed in one or more of claims 2 to 4, wherein the cultivation is carried out at 18 to 35 °C.

6. The process as claimed in one or more of claims 2 to 5, wherein the cultivation is carried out in the pH range between 6 and 8.

7. A pharmaceutical comprising the compound of the formula I as claimed in claim 1 and, where appropriate, pharmaceutically acceptable additives.

8. The use of the compound of the formula I as claimed in claim 1 for the preparation of a pharmaceutical for the prophylaxis and treatment of diseases which are based on an elevated cholesterol level.

9. The use as claimed in claim 8, wherein the compound in which R is the mannose residue is employed.

10. The use of the compound of the formula I as claimed in claim 1 for the preparation of a pharmaceutical for the prophylaxis and treatment of bacterial infections and infectious diseases.

11. Streptoverticillium sp. DSM 5989 and Streptomyces sp. DSM 5990, and the variants and mutants thereof as long as they produce the compound of the formula I as claimed in claim 1.

**Claims for the following Contracting States : ES, GR**

1. A process for the preparation of the compound of the formula I

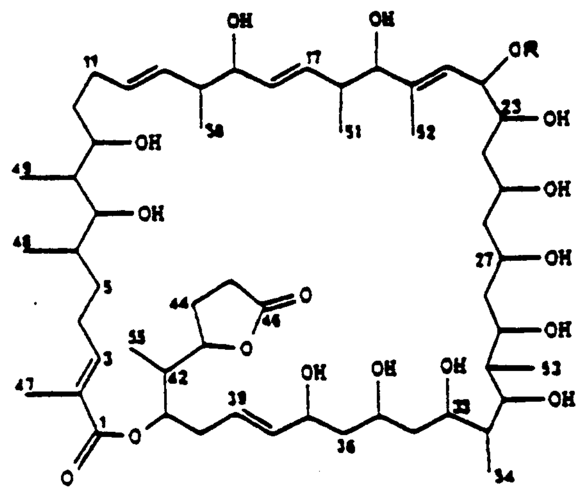

in which R is hydrogen or the group

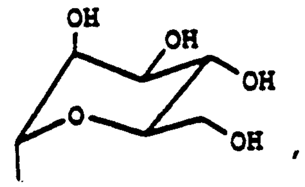

which comprises cultivating the strains Streptoverticillium sp. DSM 5989 and/or Streptomyces sp. DSM 5990 in a nutrient medium until the compound of the formula I accumulates in the culture.

2. The process as claimed in claim 1, wherein the nutrient medium contains 0.2 to 5 % soybean meal and 0.2 to 5 % mannitol, in each case based on the weight of the complete nutrient solution.

3. The process as claimed in claim 2, wherein the nutrient medium contains 0.2 to 5 % rolled oats and 0.1 to 10 ml of an aqueous trace element solution composed of 0.1 to 10 % calcium chloride, 0.01 to 5 % iron(III) citrate, 0.01 to 0.1 % manganese sulfate, 0.001 to 0.5 % zinc chloride, 0.0001 to 0.1 % copper sulfate, 0.001 to 0.5 % sodium tetraborate, 0.0001 to 0.01 % cobalt chloride and 0.0001 to 0.01 % sodium molybdate.

4. The process as claimed in one or more of claims 1 to 3, wherein the cultivation is carried out at 18 to 35 °C.

5. The process as claimed in one or more of claims 1 to 4, wherein the cultivation is carried out in the pH range between 6 and 8.

6. The use of the compound of the formula I as claimed in claim 1 for the preparation of a pharmaceutical for the prophylaxis and treatment of diseases which are based on an elevated cholesterol level.

7. The use as claimed in claim 6, wherein the compound in which R is the mannose residue is employed.

8. The use of the compound of the formula I as claimed in claim 1 for the preparation of a pharmaceutical for the prophylaxis and treatment of bacterial infections and infectious diseases.

9. Streptoverticillium sp. DSM 5989 and Streptomyces sp. DSM 5990, and the variants and mutants thereof as long as they produce the compound of the formula I as claimed in claim 1.

**Revendications**

**Revendications pour les Etats contractants suivants : AT, BE, CH, DE, DK, FR, GB, IT, LI, LU, NL, SE**

1. Compose de formule générale I

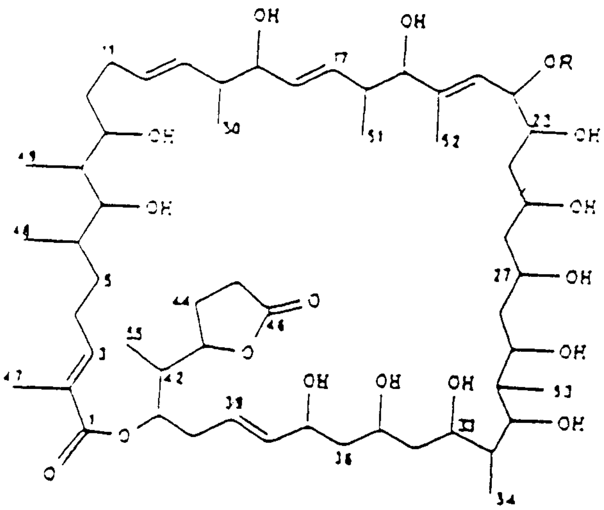

dans laquelle R représente un atome d'hydrogène ou le groupe

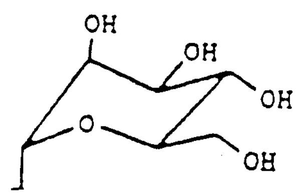

2. Procédé pour la production du composé de formule générale I selon la revendication 1, caractérisé en ce que l'on cultive dans un milieu de culture la souche *Streptoverticillium sp.* DSM 5989 et/ou la souche *Streptomyces sp.* DSM 5990, jusqu'à ce que le composé de formule générale I s'accumule dans la culture.

3. Procédé selon la revendication 2, caractérisé en ce que le milieu de culture contient de 0,2 à 5 % de farine de soja et de 0,2 à 5 % de mannitol, dans chaque cas par rapport au poids de la solution nutritive totale.

4. Procédé selon la revendication 3, caractérisé en ce que le milieu de culture contient de 0,2 à 5 % de flocons d'avoine et de 0,1 à 10 ml d'une solution aqueuse d'oligo-éléments composée de 0,1 à 10 % de chlorure de calcium, 0,01 à 5 % de citrate ferrique, 0,01 à 0,1 % de sulfate de manganèse, 0,001 à 0,5 % de chlorure de zinc, 0,0001 à 0,1 % de sulfate de cuivre, 0,001 à 0,5 % de tétraborate de sodium, 0,0001 à 0,01 % de chlorure de cobalt et 0,0001 à 0,01 % de molybdate de sodium.

5. Procédé selon une ou plusieurs des revendications 2 à 4, caractérisé en ce que la culture s'effectue à 18-35 °C.

6. Procédé selon une ou plusieurs des revendications 2 à 5, caractérisé en ce que la culture s'effectue dans l'intervalle de pH compris entre 6 et 8.

7. Médicament, contenant le composé de formule générale I selon la revendication 1 et éventuellement des additifs pharmaceutiquement acceptables.

8. Utilisation du composé de formule générale I selon la revendication 1, pour la fabrication d'un médicament destiné à la prophylaxie et au traitement de maladies qui sont dues à un taux élevé de cholestérol.

9. Utilisation selon la revendication 8, caractérisée en ce que l'on utilise le composé dans lequel R représente le reste mannose.

10. Utilisation du composé de formule générale I selon la revendication 1, pour la fabrication d'un médicament destiné à la prophylaxie et au traitement d'infections bactériennes et de maladies infectieuses.

11. *Streptoverticillium sp.* DSM 5989 et *Streptomyces sp.* DSM 5990 ainsi que leurs variants et mutants, dans la mesure où ils produisent le composé de formule générale I selon la revendication 1.

**Revendications pour les Etats contractants suivants : ES, GR**

1. Procédé pour la production du composé de formule générale I

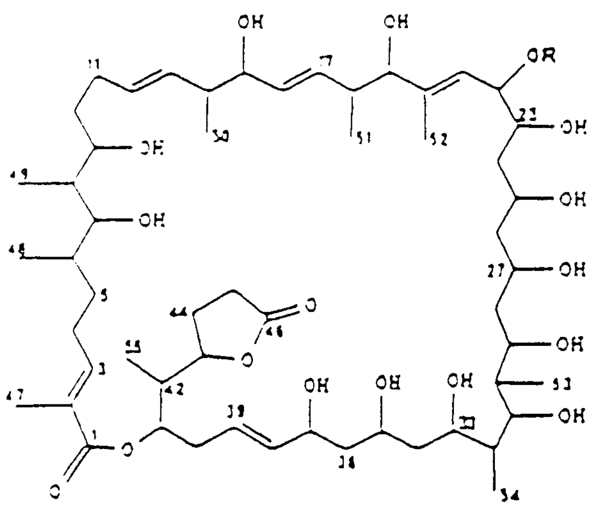

dans laquelle R représente un atome d'hydrogène ou le groupe

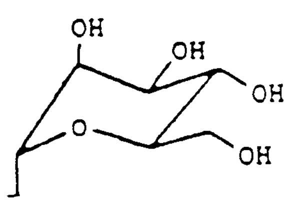

caractérisé en ce que l'on cultive dans un milieu de culture la souche *Streptoverticillium sp.* DSM 5989 et/ou la souche *Streptomyces sp.* DSM 5990, jusqu'à ce que le composé de formule générale I s'accumule dans la culture.

2. Procédé selon la revendication 1, caractérisé en ce que le milieu de culture contient de 0,2 à 5 % de farine de soja et de 0,2 à 5 % de mannitol, dans chaque cas par rapport au poids de la solution nutritive totale.

3. Procédé selon la revendication 2, caractérisé en ce que le milieu de culture contient de 0,2 à 5 % de flocons d'avoine et de 0,1 à 10 ml d'une solution aqueuse d'oligo-éléments composée de 0,1 à 10 % de chlorure de calcium, 0,01 à 5 % de citrate ferrique, 0,01 à 0,1 % de sulfate de manganèse, 0,001 à 0,5 % de chlorure de zinc, 0,0001 à 0,1 % de sulfate de cuivre, 0,001 à 0,5 % de tétraborate de sodium, 0,0001 à 0,01 % de chlorure de cobalt et 0,0001 à 0,01 % de molybdate de sodium.

4. Procédé selon une ou plusieurs des revendications 1 à 3, caractérisé en ce que la culture s'effectue à 18-35 °C.

5. Procédé selon une ou plusieurs des revendications 1 à 4, caractérisé en ce que la culture s'effectue dans l'intervalle de pH compris entre 6 et 8.

6. Utilisation du composé de formule générale I selon la revendication 1, pour la fabrication d'un médicament destiné à la prophylaxie et au traitement de maladies qui sont dues à un taux élevé de cholestérol.

7. Utilisation selon la revendication 6, caractérisée en ce que l'on utilise le composé dans lequel R représente le reste mannose.

8. Utilisation du composé de formule générale I selon la revendication 1, pour la fabrication d'un médicament destiné à la prophylaxie et au traitement d'infections bactériennes et de maladies infectieuses.

9. *Streptoverticillium sp.* DSM 5989 et *Streptomyces sp.* DSM 5990 ainsi que leurs variants et mutants, dans la mesure où ils produisent le composé de formule générale I selon la revendication 1.